Europäisches Patentamt

European Patent Office

Office européen des brevets

(19)

(11) Numéro de publication : **0 011 553**
**B1**

(12) **FASCICULE DE BREVET EUROPÉEN**

(45) Date de publication du fascicule du brevet :
13.01.82

(51) Int. Cl.³ : **C 07 C 79/04, C 07 C 76/02**

(21) Numéro de dépôt : **79400835.9**

(22) Date de dépôt : **09.11.79**

(54) **Procédé de fabrication de nitroparaffines par nitration de l'éthane en phase gazeuse.**

(30) Priorité : **14.11.78 FR 7832118**

(43) Date de publication de la demande :
**28.05.80 (Bulletin 80/11)**

(45) Mention de la délivrance du brevet :
**13.01.82 Bulletin 82/02**

(84) Etats contractants désignés :
**AT BE CH DE FR GB IT LU NL SE**

(56) Documents cités :
**DE - B - 1 069 133**
**US - A - 2 609 401**

**CHEMICAL ENGINEERING, vol. 73, 06.06.1966**
**L.F. ALBRIGHT : « Nitration of paraffins » pages 149-156**

(73) Titulaire : **SOCIETE CHIMIQUE DE LA GRANDE PAROISSE AZOTE ET PRODUITS CHIMIQUES**
**8, rue Cognacq-Jay**
**F-75007 Paris (FR)**

(72) Inventeur : **Lhonore, Pierre**
**1090, bd Jeanne d'Arc**
**F-59500 Douai (FR)**
Inventeur : **Jacquinot, Bernard**
**59, rue Léon Bathiat**
**F-59500 Douai (FR)**
Inventeur : **Quibel, Jacques**
**40, rue de la Muette**
**F-78600 Maisons Laffitte (FR)**
Inventeur : **Mari, Roger**
**8, avenue de Brabois**
**F-54600 Villers-Les-Nancy (FR)**

(74) Mandataire : **Bouton Neuvy, Liliane et al**
**L'Air liquide, Société Anonyme pour L'Etude et L'Exploitation des Procédés Georges Claude 75, Quai d'Orsay**
**F-75321 Paris Cedex 07 (FR)**

Imprimerie Jouve, 18, rue St-Denis, 75001 Paris, France

Procédé de fabrication de nitroparaffines par nitration de l'éthane en phase gazeuse

La présente invention concerne la fabrication de nitroparaffines en phase gazeuse homogène par nitration de l'éthane.

On a déjà proposé divers procédés industriels de nitration d'hydrocarbures saturés, en particulier du propane. La nitration du propane, notamment par le peroxyde d'azote, sous pression conduit à une orientation des produits finaux vers les nitropropanes avec une très nette prédominance du 2-nitropropane. Or, celui-ci ne constitue pas actuellement la nitroparaffine la plus valorisable, ni dans son emploi en tant que nitroparaffine, ni par les dérivés qu'elle permet d'obtenir.

L'un des problèmes industriels actuels réside dans la recherche des moyens permettant d'obtenir dans les opérations de nitration un spectre de produits adapté aux demandes du marché, et donc de réaliser une unité de fabrication industrielle ayant une souplesse de fonctionnement suffisante pour répondre à ces exigences. De plus, l'utilisation de matières premières les moins onéreuses et les plus disponibles en grandes quantités est également un problème industriel important. L'emploi de l'éthane est apparu comme susceptible de fournir une solution à ces deux problèmes.

Cependant, la nitration de l'éthane par les agents classiques de nitration acide nitrique et peroxyde d'azote, ne permet d'obtenir que du nitrométhane et du nitroéthane dans les proportions de 30 % de nitrométhane et 70 % de nitroéthane, ce qui ne correspond pas aux besoins du marché.

Des travaux sur la nitration de l'éthane ont été effectués à la Purdue Research Foundation dans l'Indiana aux Etats-Unis. La nitration de l'éthane a été étudiée à la pression atmosphérique par un agent nitrant tel l'acide nitrique ou le peroxyde d'azote (brevet Etats-Unis 2 071 122, et H. J. Hibshman et al. Industriel and Engineering Chemistry vol. 32 n° 3 p. 427-9). Ces expériences de laboratoires réalisées depuis plus de quarante ans ne semblent pas à ce jour avoir conduit à une réalisation industrielle du fait de la non concordance des produits fabriqués et des demandes des utilisateurs, mais aussi parce que la récupération plus difficile de l'éthane ne peut être conduite dans les mêmes conditions que pour le propane sans une dépense supérieure d'énergie, du fait du rapport molaire très élevé $C_2H_6/NO_2$.

Le brevet ETATS-UNIS 2 609 401, de H. B. Hass, de la PURDUE RESEARCH FOUNDATION a pour objet l'accroissement du taux global de conversion et du rendement en nitroparaffines et produits d'oxydation, tels alcools, aldéhydes et acides, lors de la nitration des hydrocarbures aliphatiques saturés de $C_1$ à $C_4$, en phase vapeur. L'enseignement de ce brevet met en évidence l'action positive de l'addition d'oxygène sur l'accroissement du taux global de conversion des hydrocarbures en nitroparaffines.

La publication de L. F. Albright, volume 73, 06-06-1966, dans CHEMICAL ENGINEERING montre que la poursuite des travaux de la PURDUE RESEARCH présente la température comme un facteur de la distribution des nitroparaffines fabriquées et que l'oxygène et les halogènes, tels le brome et le chlore, sont des additifs jouant un rôle dans l'accroissement de la conversion des hydrocarbures en nitroparaffines.

La DAS 1 069 133, a trait à la nitration d'alcanes lourds et de cycloalcanes en présence d'un gaz inerte.

Aucun des documents connus par l'état de la technique ne donne les moyens pour orienter la nitration de l'éthane vers une distribution prépondérante en nitrométhane.

L'invention a pour objet un procédé de fabrication industrielle de nitroparaffines à partir de l'éthane en présence d'un agent oxydant avec des rendements de conversion en nitrométhane supérieurs à ceux obtenus expérimentalement et conduisant à des teneurs élevées en nitrométhane pouvant atteindre jusqu'à 98 %.

Selon ce procédé, les rapports quantitatifs des divers constituants du mélange réactionnel, le temps de contact réactionnel, la température et la pression réactionnelles sont choisis et contrôlés de manière telle que la nitration soit mise en œuvre en phase homogène, et en fonction du spectre attendu des nitroparaffines.

La température et la pression auxquelles s'effectue la nitration sont choisies de manière telle que l'ensemble du mélange réactionnel : hydrocarbure, gaz oxygéné, agent nitrant et autres constituants éventuels, soit mis en œuvre en phase gazeuse homogène.

Les rapports des constituants du mélange réactionnel, le temps de contact et la température sont les paramètres qui permettent de faire varier le spectre de nitroparaffines produites.

L'agent nitrant est le peroxyde d'azote, l'acide nitrique, utilisés seuls ou en mélange.

L'agent oxydant est un gaz contenant de l'oxygène, tel l'air, l'air suroxygéné et l'oxygène pur.

Il a été trouvé que le spectre des nitroparaffines produites par nitration de l'éthane peut être largement modifié par addition pendant la nitration d'un agent actif porteur d'un groupement NO ou $NO_2$, facilement transférable, choisi parmi le 2-nitropropane et le nitroéthane, introduits seuls ou en mélange.

L'agent actif qui participe à la réaction est additionné à un débit tel que le rapport molaire agent actif par rapport à l'agent nitrant soit au plus égal à 3 et ajusté en fonction du spectre attendu de nitroparaffines. Les variations de ce rapport donnent une grande souplesse d'adaptation de l'installation, à la demande du marché. Et ce rapport est ajusté en fonction de la modification attendue du spectre des nitroparaffines.

L'agent actif seul ou en mélange peut très avantageusement être un produit de recyclage de la

2

réaction de nitration.

On obtient les résultats recherchés en choisissant un rapport molaire éthane/agent nitrant compris entre 12 et 2,5, de préférence entre 8 et 3 ; un rapport éthane/oxygène compris entre 12 et 45, de préférence de 15 à 40.

Le temps de contact réactionnel est fonction de la température et de la pression, compris entre 0,1 et 20 secondes et préférentiellement entre 1 à 20 secondes.

La pression de la nitration est maintenue entre 1 et 27 bars et préférentiellement entre 5 et 15 bars.

Les températures de réaction sont comprises, en ce qui concerne le peroxyde d'azote comme agent nitrant, entre 250 et 450 °C, et de préférence entre 270 et 400 °C ; et en ce qui concerne l'acide nitrique entre 340 et 600 °C, de préférence entre 420 et 520 °C.

D'autre part, il a été observé que la conduite de la nitration de l'éthane en présence d'un ou plusieurs gaz inertes, vis-à-vis de la réaction et des produits réactés, facilite la récupération de l'éthane résiduel en sortie de la nitration.

Cette possibilité de nitration est intéressante si la proportion volumétrique des gaz inertes n'excède pas 50 %, et est préférentiellement maintenue entre 7 et 30 % du volume total soumis à la nitration.

Le gaz inerte, vis-à-vis de l'ensemble des constituants réactionnels et des nitroparaffines produites, introduit seul ou en mélange, peut être choisi parmi l'azote, l'oxyde de carbone, le dioxyde de carbone, l'hydrogène, le méthane, l'argon.

Il est aussi avantageux de préchauffer les réactants à une température contrôlée au plus égale à la température de nitration. Ce préchauffage s'effectue séparément en ce qui concerne les agents oxydant et nitrant d'une part, et l'éthane d'autre part. Les gaz de recyclage ainsi que l'agent actif peuvent être préchauffés indifféremment, soit avec l'éthane, soit avec les agents oxydant et nitrant.

Le mélange réactionnel final, réalisé à température au plus égale à la température de nitration, doit être exécuté avec un soin particulier. On introduit les agents oxydant et nitrant dans le flux de l'éthane contenant éventuellement le gaz inerte. Ce mélange doit se faire en un point aussi proche que possible de la zone réactionnelle, à l'intérieur de laquelle la température doit être rigoureusement contrôlée par les moyens appropriés qui notamment permettent un bon transfert de chaleur.

Il est donné ci-après des exemples qui illustrent l'invention, à titre non limitatif.

Exemple 1

On réalise la nitration de l'éthane à des températures variant de 342 à 351 °C sous une pression de 10 bars, en présence d'air, l'agent nitrant étant le peroxyde d'azote, pendant des temps de contact de 5,8 à 7,1 secondes, les rapports molaires éthane/peroxyde d'azote en moles variant de 3,9 à 6,42 à l'entrée du réacteur, éthane/oxygène dans les mêmes conditions de 22 à 35 moles. La nitration est effectuée en présence de gaz inerte (I), constitué par de l'oxyde de carbone (essai 4), du dioxyde de carbone (essai 3), un mélange des deux oxydes de carbone (essai 1) et de l'azote (essai 2).

Les résultats sont consignés dans le tableau I. La composition de l'effluent est exprimée en pourcentage en poids de nitroparaffines et les consommations en poids de l'éthane $C_2H_6$ c et du peroxyde d'azote $NO_2$ c ramenées au kilogramme de l'ensemble des nitroparaffines fabriquées NPf.

Les résultats d'essais sont consignés dans le tableau I suivant, dans lequel les températures de réaction T°C sont en degrés centigrades, les temps de réaction ts sont exprimés en secondes, la pression P en bars, le peroxyde d'azote et l'éthane représentés par $NO_2$ et $C_2H_6$, $NO_2$ c et $C_2H_6$ représentent les consommations en poids, les nitroparaffines fabriquées sont désignées par NPf en poids, le nitrométhane par $NC_1$ et le nitroéthane par $NC_2$, les inertes sont désignés par I.

TABLEAU I

| Essai n° | 1 | 2 | 3 | 4 |
|---|---|---|---|---|
| T° C | 351 | 342 | 344 | 345 |
| TS | 7,1 | 5,9 | 5,8 | 6 |
| P bar | 10 | 10 | 10 | 10 |
| $C_2H_6/NO_2$ mol. | 5,68 | 6,42 | 3,9 | 6,4 |
| $C_2H_6/O_2$ mol. | 35 | 22 | 27 | 30 |
| Inertes | CO + $CO_2$ | $N_2$ | $CO_2$ | CO |
| $I/C_2H_6$ mol. | CO : 0,33<br>$CO_2$ : 0,34 | 0,17 | 0,31 | 0,16 |

3

TABLEAU I (suite)

| Essai n° | 1 | 2 | 3 | 4 |
|---|---|---|---|---|
| Composition NP | | | | |
| 2 $NC_3$ % | 7 | | | |
| $NC_1$ % | 35 | 30 | 31 | 30 |
| $NC_2$ % | 58 | 70 | 69 | 70 |
| $\dfrac{C_2H_6\ c}{NPf}$ | 4,98 | 1,89 | 0,73 | 1,88 |
| $\dfrac{NO_2\ c}{NPf}$ | 3,17 | 1,48 | 1,18 | 1,33 |

## Exemple 2

On réalise la nitration de l'éthane à des températures variant de 326 à 353 °C, sous une pression de 10 bars, en présence d'air, l'agent nitrant étant le peroxyde d'azote, pendant des temps de contact variant de 6 à 7,2 secondes, les rapports molaires éthane/peroxyde d'azote variant de 3,8 à 7,92 moles et éthane/oxygène dans les mêmes conditions de 21,7 à 35 moles. La nitration est effectuée en présence d'un agent actif : (A-A) : nitropropane ou nitroéthane de recyclage, ainsi qu'en présence de gaz inerte.

Les résultats d'essais sont consignés dans le tableau II.

La consommation spécifique de l'agent actif recyclé (AARC) ou (introduit) représente la différence entre la masse introduite et la masse du même constituant recueillie en sortie du réacteur. Elle est exprimée en kg du corps recyclé par kg de nitroparaffines produites NPf. Dans tous les essais où la réaction de nitration est conduite avec recyclage ou introduction d'un agent actif, pour la détermination de la composition de ce corps dans les liquides fabriqués, on ne tient compte que de la différence entre la masse de ce composé recueillie et la masse de ce même composé actif introduite à l'entrée du réacteur.

TABLEAU II

| Essai n° | 5 | 6 | 7 | 8 | 9 | 10 |
|---|---|---|---|---|---|---|
| T° C | 345 | 342 | 350 | 353 | 326 | 344 |
| TS | 7,2 | 7,1 | 7 | 6,1 | 6 | 6 |
| P bar | 10 | 10 | 10 | 10 | 10 | 10 |
| $C_2H_6/NO_2$ mol. | 5,7 | 5,7 | 5,7 | 7,92 | 3,8 | 3,8 |
| $C_2H_6/O_2$ mol. | 35 | 35 | 35 | 21,7 | 25,1 | 25,1 |
| Inerte $I/C_2H_6$ mol. | $CO_2$ 0,64 | CO 0,67 | $CO + CO_2$ CO : 0,33 $CO_2$ : 0,34 | $N_2$ 0,17 | $CO_2$ 0,31 | $CO_2$ 0,31 |
| Recyclage $AA/NO_2$ mol. | 2 $NC_3$ 0,13 | 2 $NC_3$ 0,15 | 2 $NC_3$ 0,16 | $NC_2$ 0,17 | $NC_2$ 0,12 | $NC_2$ 0,11 |
| Composition NP | | | | | | |
| $NC_1$ % | 37,4 | 35,4 | 37,6 | 59 | 49,3 | 98 |
| $NC_2$ % | 62,6 | 64,6 | 62,4 | 38 | 50,7 | 2 |
| 2 NC 3 % | | | | 3 | | |

## 0 011 553

TABLEAU II (suite)

| Essai n° | 5 | 6 | 7 | 8 | 9 | 10 |
|---|---|---|---|---|---|---|
| $\dfrac{C_2H_6\ c}{NP\ f}$ | 1,97 | 1,60 | 4,92 | 1,80 | 1,47 | 3,66 |
| $\dfrac{NO_2\ c}{NP\ f}$ | 1,88 | 1,44 | 3,17 | 3,21 | 1,90 | 1,16 |
| $\dfrac{AAR\ c}{NP\ f}$ | 0,55 | 0,72 | 0,79 | | | 0,19 |

### Exemple 3

On réalise la nitration de l'éthane à des températures de 338 et 341 °C, sous une pression de 10 bars, l'agent nitrant étant le peroxyde d'azote, pendant des temps de contact de 6 et 6,1 secondes, les rapports molaires $C_2H_6/NO_2$ mol. étant de 7,54 et 7,77 et les rapports molaires $C_2H_6/O_2$ mol. étant de 21 et 22. La nitration est conduite en présence de nitroéthane de recyclage $NC_2R$, en absence d'inerte.

Les résultats sont consignés dans le tableau III ci-après.

TABLEAU III

| Essai n° | 11 | 12 |
|---|---|---|
| T° C | 341 | 338 |
| TS | 6,1 | 6 |
| P bar | 10 | 10 |
| $C_2H_6/NO_2$ mol. | 7,54 | 7,77 |
| $C_2H_6/O_2$ mol. | 21 | 22 |
| N $C_2R/NO_2$ mol. | 0,20 | 0,15 |
| Composition NP<br>N $C_1$ %<br>N $C_2$ %<br>2 N $C_3$ % | 46<br>51<br>3 | 88<br>9<br>3 |
| $C_2H_6$ c/NP f | 1,08 | 2,16 |
| $NO_2$ c/NP f | 2,11 | 1,26 |

### Revendications

1. Procédé de fabrication de nitroparaffines à teneur élevée en nitrométhane par nitration en phase homogène de l'éthane par un agent nitrant tels le peroxyde d'azote, l'acide nitrique seuls ou en mélange, en présence d'un agent oxydant, caractérisé en ce que la réaction de nitration est conduite en présence d'un agent actif choisi parmi le 2-nitropropane et le nitroéthane introduits seuls ou en mélange dans un rapport molaire agent actif/agent nitrant au plus égal à 3 ajusté en fonction du spectre attendu de nitroparaffines.

2. Procédé de fabrication de nitroparaffines par nitration en phase homogène de l'éthane selon la revendication 1, caractérisé en ce que l'agent actif est un produit de recyclage de la réaction.

3. Procédé de fabrication de nitroparaffines par nitration en phase homogène de l'éthane selon la revendication 1, caractérisé en ce que la réaction est conduite en outre en présence d'au moins un gaz

5

**0 011 553**

inerte, vis-à-vis de l'ensemble des constituants réactionnels et des nitroparaffines produites, choisi parmi l'azote, l'oxyde de carbone et le dioxyde de carbone seuls ou en mélange, introduit dans une proportion volumétrique n'excédant pas 50 % du volume total soumis à la nitration, de préférence 7 à 30 %.

4. Procédé de fabrication de nitroparaffines par nitration en phase homogène de l'éthane selon la revendication 1, caractérisé en ce que le rapport molaire éthane/agent nitrant est compris entre 12 et 2,5 ; le rapport molaire éthane/oxygène étant compris entre 12 et 45 ; le temps de contact réactionnel est compris entre 0,1 et 20 secondes ; la pression réactionnelle comprise entre 1 et 27 bars, et la température réactionnelle est comprise entre 250 et 450 °C quand l'agent nitrant est le peroxyde d'azote.

5. Procédé de fabrication de nitroparaffines par nitration en phase homogène de l'éthane selon la revendication 1, caractérisé en ce que le rapport molaire éthane/agent nitrant est compris entre 8 et 3, le rapport molaire éthane/ oxygène est compris entre 15 et 40, le temps de contact réactionnel est compris entre 1 et 10 secondes, la pression réactionnelle entre 5 et 15 bars ; et la température réactionnelle entre 270 et 400 °C quand l'agent est le peroxyde d'azote.

## Claims

1. Process for the production of nitro-paraffins having a high proportion of nitromethane by nitration of ethane in the homogenous phase using a nitrating agent such as nitrogen peroxide or nitric acid alone or in admixture in the presence of an oxidising agent, characterised in that the nitration reaction is carried out in the presence of an active agent selected from 2-nitropropane and nitro-ethane fed in singly or in admixture in a molar ratio of active agent to nitrating agent of not more than 3 and correlated as a function of the nitroparaffins to be produced.

2. Process for the production of nitro-paraffins by nitration of ethane in the homogenous phase according to Claim 1, characterised in that the active agent is a recycled reaction product.

3. Process for the production of nitro-paraffins by nitration of ethane in the homogenous phase according to Claim 1, characterised in that the reaction is inter alia carried out in the presence of at least one gas which is inert with respect to all the reactive ingredients and to the nitro-paraffins which are produced, selected from nitrogen, carbon monoxide and carbon dioxide, either alone or in admixture, fed in a volumetric fraction not exceeding 50 % of the total volume submitted to nitration, preferably from 7 to 30 % thereof.

4. Process for the production of nitro-paraffins by nitration of ethane in the homogenous phase according to Claim 1, characterised in that the molar ratio of ethane to nitrating agent is between 12 and 2.5 ; the molar ratio of ethane to oxygen is between 12 and 45 ; the reaction contact period is between 0.1 and 20 seconds ; the reaction pressure is between 1 and 27 bars and the reaction temperature is between 250 and 450 °C when the nitrating agent is nitrogen peroxide.

5. Process for the production of nitro-paraffins by nitration of ethane in the homogenous phase according to Claim 1, characterised in that the molar ratio of ethane to nitrating agent is between 8 and 3, the molar ratio of ethane to oxygen is between 15 and 40, the reaction contact period is between 1 and 10 seconds, the reaction pressure between 5 and 15 bars, and the reaction temperature between 270 and 400 °C when the agent is nitrogen peroxide.

## Ansprüche

1. Verfahren zur Herstellung von Nitroparaffinen mit hohem Nitromethangehalt durch Nitrierung von Äthan in homogener Phase mit einem Nitriermittel, wie Stickstoffperoxid und/oder Salpetersäure, in Gegenwart eines Oxidationsmittels, dadurch gekennzeichnet, daß die Nitrierreaktion in Gegenwart eines aktiven Mittels aus der Gruppe 2-Nitropropan und/oder Nitroäthan durchgeführt wird, welche in einem Molverhältnis von aktivem Mittel zu Nitriermittel höchstens gleich 3, eingestellt in Funktion des erwarteten Spektrums der Nitroparaffine, eingeführt werden.

2. Verfahren zur Herstellung von Nitroparaffinen durch Nitrierung von Äthan in homogener Phase nach Anspruch 1, dadurch gekennzeichnet, daß das aktive Mittel ein Rezyklierungsprodukt der Reaktion ist.

3. Verfahren zur Herstellung von Nitroparaffinen durch Nitrierung von Äthan in homogener Phase nach Anspruch 1, dadurch gekennzeichnet, daß die Reaktion außerdem in Gegenwart wenigstens eines gegenüber der Gesamtheit der reaktionsfähigen Bestandteile und der erzeugten Nitroparaffine inerten Gases aus der Gruppe Stickstoff, Kohlenmonoxid und/oder Kohlendioxid durchgeführt wird, welches in einem Volumenverhältnis eingeführt wird, das 50 % des der Nitrierung unterzogenen Gesamtvolumens nicht überschreitet, vorzugsweise bei 7 bis 30 % liegt.

4. Verfahren zur Herstellung von Nitroparaffinen durch Nitrierung von Äthan in homogener Phase nach Anspruch 1, dadurch gekennzeichnet, daß das Molverhältnis von Äthan zu Nitriermittel zwischen 12 und 2,5, das Molverhältnis von Äthan zu Sauerstoff zwischen 12 und 45, die Reaktionskontaktzeit zwischen 0,1 und 20 Sekunden, der Reaktionsdruck zwischen 1 und 27 bar und die Reaktionstemperatur zwischen 250 und 450 °C liegt, wenn das Nitriermittel Stickstoffperoxid ist.

6

5. Verfahren zur Herstellung von Nitroparaffinen durch Nitrierung von Äthan in homogener Phase nach Anspruch 1, dadurch gekennzeichnet, daß das Molverhältnis von Äthan zu Nitriermittel zwischen 8 und 3, das Molverhältnis von Äthan zu Sauerstoff zwischen 15 und 40, die Reaktionskontaktzeit zwischen 1 und 10 Sekunden, der Reaktionsdruck zwischen 5 und 15 bar und die Reaktionstemperatur zwischen 270 und 400 °C liegt, wenn das Mittel Stickstoffperoxid ist.